# EUROPEAN PATENT SPECIFICATION

(11) **EP 3 541 291 B1**
(45) Date of publication and mention of the grant of the patent: **11.08.2021**
(21) Application number: 17809196.3
(22) Date of filing: 09.11.2017
(51) Int. Cl.: A61B 8/08, A61B 8/00

(54) **REMOTE ULTRASONIC DIAGNOSIS WITH CONTROLLED IMAGE DISPLAY QUALITY**
ENTFERNTE ULTRASCHALLDIAGNOSE MIT KONTROLLIERTER BILDANZEIGEQUALITÄT
DIAGNOSTIC ULTRASONORE À DISTANCE À QUALITÉ D'AFFICHAGE D'IMAGE COMMANDÉE

(30) Priority: 17.11.2016 US 201662423477 P
(43) Date of publication of application: 25.09.2019
(73) Proprietor: Koninklijke Philips N.V., 5656 AG Eindhoven (NL)
(72) Inventor: RAJU, Balasundar Iyyavu, 5656 AG Eindhoven (NL); HEGDE, Sanjay Ramachandra, 5656 AG Eindhoven (NL)
(74) Representative: Philips Intellectual Property & Standards
(86) International application number: PCT/EP2017/078678
(87) International publication number: WO 2018/091337

(56) References cited:
- EP-A2- 2 892 024
- WO-A1-2016/028787
- KR-A- 20120 124 123
- US-A1- 2013 116 563

## Description

This invention relates to medical diagnostic ultrasound systems and, in particular, to the use of ultrasound systems for remote diagnosis with control of the local image display quality.

Currently available medical ultrasound systems enable clinicians to conduct ultrasound scans on a patient, capture images, make measurements and use built-in algorithms and report generation software to make diagnoses and report the results of a diagnosis. The clinicians who perform these procedures are experienced radiologists, cardiologists, obstetricians, or trained sonographers. Sufficient training is expected before a clinician can conduct the procedure and interpret the scanned images. However, such medical experts may not be readily available in a remote area. One approach to providing necessary diagnostic care is teleradiology, whereby an inexperienced person at the location of the patient is guided in the conduct of a scan by a remotely located medical expert. But providing remote assistance with the help of a local user (e.g., the patient himself/herself) has to be robust and secure enough to meet appropriate security guidelines. It should also be a low cost solution so that patients and other inexperienced persons are encouraged to conduct the scan.

Moreover, many countries have laws in place to restrict the use of ultrasound for use by an expert for medical diagnosis purpose only. In some countries gender identification is an issue and it is illegal to determine gender of a fetus before birth using ultrasound. Only licensed persons can perform ultrasound imaging. If a remote ultrasound system is provided to the patient for legitimate use such as checking the condition of the heart, it is conceivable that the device can be misused for gender identification purposes. One approach to preventing such misuse is to block the display of images to unqualified or unauthorized users. However, preventing display of the image locally to the user may make it difficult to perform a useful lawful scan, as the user may need some guidance in probe placement which only an image can provide.

US 2013/116563 discloses an ultrasonic apparatus having a first ultrasound image generation unit and a second ultrasound image generation unit, wherein the first unit is adapted to generate a first image at a first processing speed, and the second unit is adapted to generate second image at a second processing speed, faster than the first processing speed, the second image having larger data than the first image. KR 2012 0124123 discloses a smart phone controlled ultrasonic probe for use as an ultrasonic diagnostic equipment.

The invention is defined by the claims.

Accordingly, it is an object of the present invention to make ultrasound imaging available at the site of a patient with the help of an expert at a remote location. It is a further object to do so in a way that facilitates local scanning with remote expert assistance without also facilitating improper use of the scanning device such as unlawful gender identification. A solution which meets these objectives will enable diagnostic imaging in areas where diagnostic imaging experts are not readily available for cost or other reasons.

In some aspects, the present invention provides a system for remote guidance of a scanning procedure. The system can include a local ultrasound scanning device comprising an ultrasound probe, an image display, and an image communication unit adapted to send images to and receive control signals from a remote location. The system can also include an ultrasound system that is located at the remote location and adapted to display images received from the local ultrasound scanning device and send control signals to the local ultrasound scanning device. The local ultrasound scanning device can include an image processor that is responsive to control signals received from the ultrasound system and adapted to produce low image quality images for display on the image display and high quality images for sending to the ultrasound system at the remote location, and the image display can be configured to display the lower image quality images of the local ultrasound scanning device.

In certain aspects, the low image quality images comprise a lower image resolution than the high quality images. Quality factors for the low quality images and the high quality images can be set according to a default setting in the local ultrasound scanning device. Alternatively, quality factors for the low quality images and the high quality images can be set according to an image quality control signal received from the ultrasound system located at the remote location. In some aspects, the ultrasound probe can be adapted for scanning a patient at the site of the local ultrasound scanning device under guidance from an expert at the remote location. The image processor of the local ultrasound scanning device can include a scan converter configured to produce the high quality images with full interpolation of image pixel values between echo pixels and low quality images having no interpolated values added to the echo pixels. The image processor of the local ultrasound scanning device further can include a video compression processor. In certain aspects, the local ultrasound scanning device and the ultrasound system each include a network communication unit adapted to send and receive control and or image data.

The present invention also includes methods for guiding an ultrasonic scanning procedure from a remote location. The method can include ultrasonically scanning a subject with an ultrasound scanning device at a patient site, sending high quality ultrasound images acquired by the ultrasonic scanning to a remote site, producing low quality ultrasound images in response to an image quality control signal or a default setting on the ultrasound scanning device, and displaying low quality ultrasound images at the patient site.

The methods can also include providing scanning guidance to the patient site from the remote site, displaying the high quality ultrasound images on an image display device at the remote site, making a diagnosis using the high quality ultrasound images at the remote site, producing low quality ultrasound images is performed by the ultrasound scanning device, establishing voice communication between the patient site and the remote site, and/or enabling the ultrasound scanning device for scanning from the remote site.

In the drawings:
FIGURE 1 illustrates an ultrasound scanning device and a remote expert diagnostic workstation configured in accordance with the principles of the present invention.
FIGURE 2 illustrates in block diagram form an ultrasonic scanning device configured for use by an untrained user in accordance with the principles of the present invention.
FIGURE 3 is a flowchart illustrating the interaction of an ultrasonic scanning device and a remotely located expert in accordance with the present invention.

In accordance with the principles of the present invention a system and method are described which enable ultrasonic imaging by inexperienced personnel under the guidance of a remotely located diagnostic imaging expert. Improper use of the scanning device is prevented by controlling the device to present only low quality images to the person conducting the scan, while images of high quality are sent to the remote expert for diagnosis. Various ways are described for controllably setting the quality of the ultrasound images presented to the inexperienced person conducting the scan. Thus, an effective diagnosis by the expert is facilitated while providing at the scanning site only images which, while sufficient to provide visual guidance of the scanning procedure, cannot be used improperly by persons at the site of the patient being scanned.

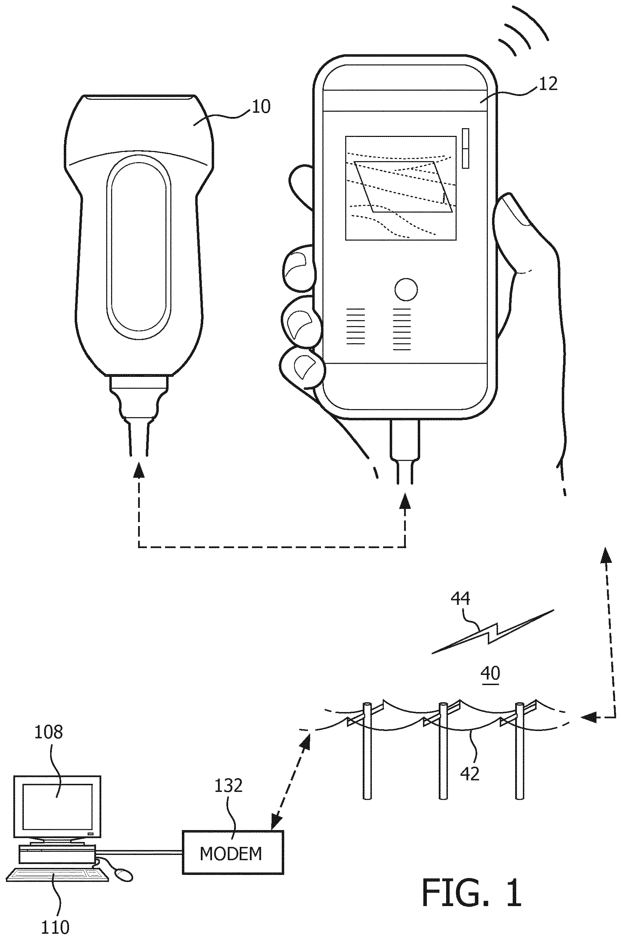

FIGURE 1 illustrates at the top of the drawing an easy-to-use ultrasonic scanning device comprising an L12-5 Lumify™ ultrasound probe 10 coupled to a handheld display device in the form of a smartphone 12. The Lumify ultrasound system, available from Philips Healthcare of Andover, MA, USA, consists of a Lumify probe such as the one shown in the drawing, and an image display device, which may comprise a PDA, laptop computer, tablet computer, or smartphone 12 as shown in this illustration. In this system all of the ultrasound-specific components, such as the transducer array, beamformer, ultrasound signal processor, and B mode and Doppler processors, are constructed in piezoelectronic and integrated circuit form and located in the probe 10. The display device 12 contains the image display and the user interface components (in the case of a smartphone, in touchscreen form). The display device also contains a software application specific to the Lumify probe, which causes the ultrasound image and the user controls to be displayed on the device screen as shown in the illustration. In this smartphone implementation the user holds the ultrasound probe 10 against the skin of the patient with one hand while holding the display and user control device 12 with the other hand. The acquired ultrasound images are displayed on the display device with an image quality set by a remotely located expert as described below, and the same images in high quality form are sent wirelessly to the display device of the remote expert. In the illustrated implementation the expert's ultrasound image display device is the screen of a workstation 108 which is controlled by a keyboard 110. The expert sends commands to the scanning device and receives images from the scanning device by means of a modem or wireless radio 132 which are carried over a data network 40, e.g., the Internet, which may take the form of a wire network 42 or a wireless network as indicated by 44.

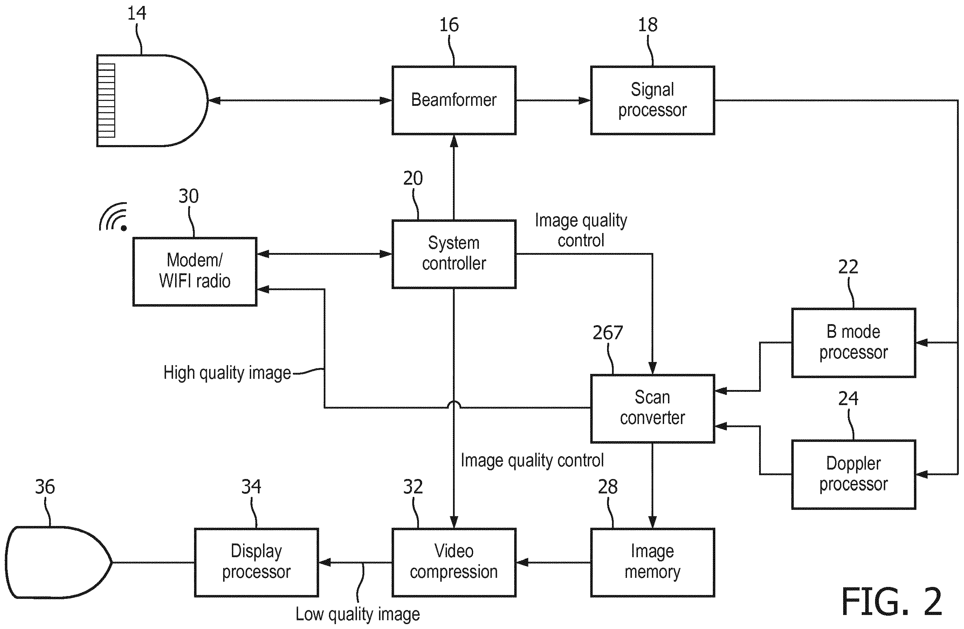

FIGURE 2 illustrates the components of an exemplary ultrasonic scanning device in block diagram form. An ultrasonic probe such as 10 in FIGURE 1 includes an array 14 of ultrasonic transducer elements that transmit and receive ultrasonic pulses. The array may be a one dimensional linear or curved array for two-dimensional imaging, or may be a two-dimensional matrix of transducer elements for electronic beam steering and focusing in two or three dimensions. The ultrasonic transducer elements in the array 14 transmit beams of ultrasonic energy by their timed actuation by a beamformer 16, and receive echoes returned in response to this transmission.

Echoes from the transmitted ultrasonic energy are received by the transducers of the array 14, which generate echo signals that are coupled to the beamformer 16 where they are digitized and beamformed into coherent echo signals. The echo signal samples from the individual transducer elements of the array 14 are delayed and summed by the beamformer 16 to form coherent echo signals along scanline directions for an image. The coherent echo signals undergo signal processing by a signal processor 18, which include filtering by a digital filter and noise reduction as by spatial or frequency compounding.

The signal processor can also shift the frequency band to a lower or baseband frequency range. The digital filter of the signal processor 18 can be a filter of the type disclosed in U.S. Patent No. 5,833,613 (Averkiou et al.), for example. When phase information is needed as is the case for Doppler processing, quadrature (I and Q) demodulation is also performed by the signal processor.

The beamformed and processed coherent echo signals are coupled to a B mode processor 22 which produces a B mode tissue image. The B mode processor performs amplitude (envelope) detection of quadrature demodulated I and Q signal components by calculating the echo signal amplitude in the form of (I²+Q²)^{½}.
The quadrature echo signal components are also coupled to a Doppler processor 24, which stores ensembles of echo signals from discrete points in an image field which are then used to estimate the Doppler shift at points in the image with a fast Fourier transform (FFT) processor. For a color Doppler image such as that shown on the display device in FIGURE 1, the estimated Doppler flow values at each point in a blood vessel are wall filtered and converted to color values using a look-up table. The B mode image signals and the Doppler flow values are coupled to a scan converter 26 which converts the B mode and Doppler samples from their acquired R-θ coordinates to Cartesian (x,y) coordinates for display in a desired display format, e.g., a sector format or a rectilinear image format as shown in FIGURE 1. Either the B mode image or the Doppler image may be displayed alone, or the two shown together in anatomical registration in which the color Doppler overlay shows the blood flow in tissue and vessels in the image.

The pixel density a typical image display is generally greater than the density of echo signals acquired from an image field of the body. Accordingly, scan conversion also typically includes calculating additional image points for pixel display between the image points of acquired echo signals. The spaces between acquired scanlines and echo signals are usually filled in by four-point interpolation of the echo signals surrounding a void in the display field. In accordance with the principles of the present invention, in one implementation the scan converter 26 produces two images of each scene in the image field with two different display qualities. One way to do this is to produce the usual scan converted image with full interpolation of image pixel values between echo pixels, and a second, low quality image in which no interpolated values are added to the echo pixels.
The latter image will thus be coarse with poor anatomical resolution. The poorly resolved image is stored in an image memory 28, from which it is coupled to a display processor 34 for display on the image display 36 of the scanning device as shown in FIGURE 1. The normal, high quality image is coupled to a network communication unit such as a modem or WiFi radio 30, which transmits the image to the display device of the remotely located expert. Thus, the remotely located expert will see a high resolution, high quality image which is suitable for making a diagnosis of the patient's condition, while the individuals located at the site of the patient, the untrained person conducting the scan and/or the patient, will see the same image but of nondiagnostic quality and poor resolution. The locally displayed image, while sufficient to provide guidance of the scan, is of low enough quality that unintended uses of it such as gender identification cannot be performed with the image.

In some aspects, the locally displayed image can have image quality factors that are set according to a default setting on the local ultrasound system.
For example, the default setting can include poor image resolution, or other quality parameters such as gain or depth, that are of lower quality than that at the remote imaging system or workstation. In other aspects, the degree of the resolution and/or image quality parameters of the locally displayed image can be set by the remote expert communicating with his or her imaging system or workstation. Prior to scanning at the patient site, the expert sends an image quality control signal to the scanning system, which is received by the modem/WiFi radio 30 and coupled to a system controller 20. The system controller then couples an image quality control signal to the scan converter 26, instructing the scan converter how to form the low quality image. In the foregoing example, this was done by instructing the scan converter to fill in none of the voids between acquired echo signal image points with smoothed, interpolated values, thereby producing a coarse, low quality image for local display. Other approaches may also be taken, such as injecting noise into the image, as by adding a small random number to each display value in the image. Spatial or temporal filtering may also be employed to degrade the quality of an image. Image modification techniques which blur an image may also be used.

FIGURE 2 illustrates yet another approach for producing a low quality image on the local display 36, which is through control of video compression.
In this implementation the usual high quality scan converted image is coupled to the modem/WiFi radio for communication to the expert and is also stored in the image memory 28. Before the image is displayed on the local display 36, it undergoes controllable video compression by a video compression processor 32. Video compression techniques such as JPEG and MPEG compression are well known in the video processing art. By applying a high degree of video compression, the image quality is degraded so that the ultrasound image is no longer usable diagnostically. The degree of video compression applied is controlled by an image quality control signal provided by the system controller 20 in response to reception of an image quality control signal from the diagnostic expert's system. The resultant low quality image is coupled to the display processor 34 for display on the local image display 36. As in the case of the previous examples, the remote expert using the images diagnostically sees high, diagnostic quality images, whereas persons at the patient site see only images of low image quality.

In the Lumify system of FIGURE 1, the display processor 34 and the display 36, and the modem/WiFi radio 30 are locate in the smartphone display unit 12, while the other components of FIGURE 2 are located in the probe 10. The user controls on the touchscreen display of the smartphone display unit, not shown in FIGURE 2, send control signals to the system controller 20, which appropriately controls the other components of the scanning system in accordance with the user's manipulation of the controls. Other implementations, for instance, ones using a tablet or laptop computer as the display device, may partition the components differently so that more components are located in the computer.
The video compression 32, for example, may be performed by the computer, as well as the image memory storage and other image processing functions.

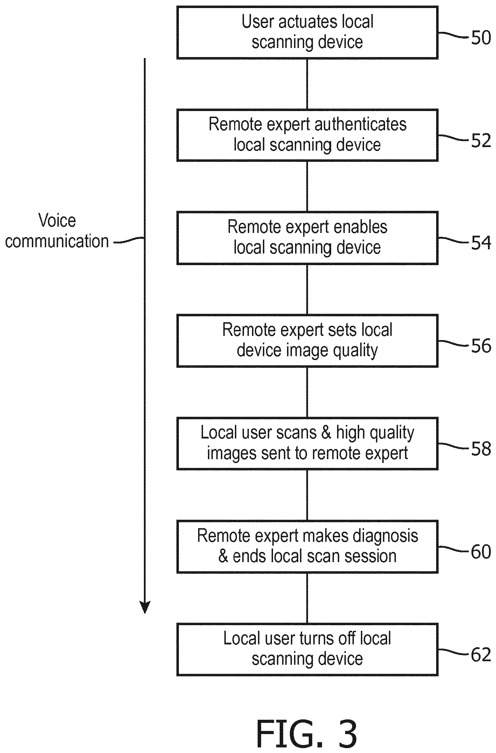

A method for use of the ultrasound scanning device of FIGURE 1 by an untrained user and the remote image workstation of the drawing by a diagnosing expert is outlined by the flowchart of FIGURE 3. When a person at the patient site, such as the patient himself or herself, is ready to conduct a scan, this user actuates the ultrasonic scanning device at 50, as by turning it on. The steps of FIGURE 3 are generally accompanied by voice communication between the user and the expert, as by telephone or voice-over-Internet use of the (e.g., smartphone) scanning device. Actuating the scanning device sends a request for a scan procedure to the workstation or imaging system of a remotely located expert such as a trained physician or sonographer. After the remote expert has determined that the scanning request is valid, the expert sends a signal authenticating the local scanning device in step 52. When the remote expert is ready to assist the procedure, he or she enables the local scanning device at 54 and sends a command to the scanning device at 56 which sets the image quality of the local display 36. This command is used to set the local image quality using, for instance, the image resolution setting techniques described above. At step 58 the untrained local user begins scanning with the scanning device and high quality images are sent to the remote expert for review on the expert's display 108. The local user sees only images of low quality as set by the expert at 56. As the local user scans, he or she receives oral guidance from the voice communication with the expert, instructing the user in actions such as how to hold the probe 10 or position the probe so as to acquire an image of the necessary image plane for a diagnostic image. The local user also can see the results of his or her actions in the changes of the low quality image shown on the local display 36. When the local user is acquiring images of the proper view of anatomy for a diagnosis, the remotely located expert makes a diagnosis of the patient condition and ends the local scanning session at 60. In concluding the scanning session, the expert may command the scanning device as to which, if any, images are to be stored on the scanning device and the image quality in which they are to be stored. The results of the diagnosis may be communicated to the patient or medical personnel at the patient location for follow-on care or treatment as necessary. With the scan session ended, the local user turns off the local scanning device at 62.

Variations of the system and method described above will readily occur to those skilled in the art. The system used by the expert in FIGURE 1 can be a fully functional ultrasound diagnostic imaging system instead of a workstation, for instance. The display of images at the scanning location may be delayed in time relative to their display at the expert's location, giving the expert time to see the images first and block the display of any images that may be deemed inappropriate at the scanning site.

It should be noted that an ultrasound system suitable for use in an implementation of the present invention, and in particular the component structure of the workstation and ultrasound systems of FIGURES 1 and 2, may be implemented in hardware, software or a combination thereof. The various embodiments and/or components of an ultrasound system, for example, the modules, or components and controllers therein, also may be implemented as part of one or more computers or microprocessors. The computer or processor may include a computing device, an input device, a display unit and an interface, for example, for accessing the Internet as shown in FIGURE 1. The computer or processor may include a microprocessor. The microprocessor may be connected to a communication bus, for example, to access a PACS system or the data network shown in FIGURE 1. The computer or processor may also include a memory. The memory devices such as the image memory 28 may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and transmission of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine. In the Lumify system smartphone shown in FIGURE 1, for instance, software instructions are conventionally employed to create and control the display and user control functions described above.

## Claims

1. A system for remote guidance of a scanning procedure comprising:
a local ultrasound scanning device comprising an ultrasound probe (10), an image display (12), and an image communication unit (30) adapted to send images to and receive control signals from a remote location;
an ultrasound system (108) that is located at the remote location and adapted to display images received from the local ultrasound scanning device and send control signals to the local ultrasound scanning device;
wherein the local ultrasound scanning device further comprises an image processor that is responsive to control signals received from the ultrasound system and adapted to produce low image quality images for display on the image display and high quality images for sending to the ultrasound system at the remote location,
wherein the image display is configured to display the lower image quality images of the local ultrasound scanning device.

2. The system of any one of the claims above, wherein the low image quality images comprise a lower image resolution than the high quality images.

3. The system of any one of the claims above, wherein the quality factors for the low quality images and the high quality images are set according to a default setting in the local ultrasound scanning device.

4. The system of any one of the claims above, wherein quality factors for the low quality images and the high quality images are set according to an image quality control signal received from the ultrasound system located at the remote location.

5. The system of any one of the claims above, wherein the local ultrasound scanning device is adapted to receive guidance from an expert at the remote location.

6. The system of any one of the claims above, wherein the image processor of the local ultrasound scanning device comprises a scan converter configured to produce the high quality images with full interpolation of image pixel values between echo pixels and low quality images having no interpolated values added to the echo pixels.

7. The system of any one of the claims above, wherein the image processor of the local ultrasound scanning device further comprises a video compression processor.

8. The system of any one of the claims above, wherein the local ultrasound scanning device and the ultrasound system each include a network communication unit adapted to send and receive control and or image data.

9. A method for guiding an ultrasonic scanning procedure from a remote location comprising:
ultrasonically scanning a subject with an ultrasound scanning device at a patient site;
sending high quality ultrasound images acquired by the ultrasonic scanning to the remote site;
producing low quality ultrasound images in response to an image quality control signal received from the remote location or a default setting on the ultrasound scanning device; and
displaying the low quality ultrasound images at the patient site.

10. The method of any one of the method claims above, comprising obtaining scanning guidance at the remote site and providing the scanning guidance to the patient site.

11. The method of any one of the method claims above, comprising displaying the high quality ultrasound images on an image display device at the remote site.

12. The method of any one of the method claims above, wherein producing low quality ultrasound images is performed by the ultrasound scanning device.

13. The method of any one of the method claims above, comprising establishing voice communication between the patient site and the remote site.

14. The method of any one of the method claims above, comprising enabling the ultrasound scanning device for scanning from the remote site.

## Patentansprüche

1. Ein System zur Fernsteuerung eines Abtastvorgangs, das Folgendes umfasst:
ein lokales Ultraschall-Abtastgerät, das eine Ultraschallsonde (10), eine Bildanzeige (12) und eine Bildkommunikationseinheit (30) umfasst und
Bilder an einen Remote-Standort sendet und Steuerungssignale von dort empfängt;
ein Ultraschallsystem (108),
das sich an dem Remote-Standort befindet und die vom lokalen Ultraschall-Abtastgerät abgerufenen Bilder anzeigt und Steuerungssignale an das lokale Ultraschall-Abtastgerät sendet;
wobei das lokale Ultraschall-Abtastgerät zudem einen Bildprozessor umfasst, der auf die vom Ultraschallsystem abgerufenen Steuersignale anspricht und Bilder mit niedriger Bildqualität für die Anzeige auf der Bildanzeige sowie Bilder mit hoher Bildqualität zum Senden an das Ultraschallsystem am Remote-Standort erzeugt,
wobei die Bildanzeige die Bilder mit niedrigerer Bildqualität vom lokalen Ultraschall-Abtastgerät anzeigt.

2. Das System gemäß einem der vorherigen Ansprüche, wobei die Bilder mit niedriger Bildqualität eine geringere Bildauflösung als die Bilder mit hoher Bildqualität aufweisen.

3. Das System gemäß einem der vorherigen Ansprüche, wobei die Qualitätsfaktoren für die Bilder mit niedriger Bildqualität und die Bilder mit hoher Bildqualität auf dem lokalen Ultraschall-Abtastgerät voreingestellt sind.

4. Das System gemäß einem der vorherigen Ansprüche, wobei die Qualitätsfaktoren für die Bilder niedriger Qualität und die Bilder hoher Qualität anhand eines Bildqualitätssteuerungssignal eingestellt werden, das vom Ultraschallsystem am Remote-Standort abgerufen wird.

5. Das System gemäß einem der vorherigen Ansprüche, wobei das lokale Ultraschall-Abtastgerät eine Anleitung von einem Experten am Remote-Standort abruft.

6. Das System gemäß einem der vorherigen Ansprüche, wobei der Bildprozessor des lokalen Ultraschall-Abtastgeräts einen Abtastwandler umfasst, der die Bilder mit hoher Qualität mit vollständiger Interpolation der Bildpixelwerte zwischen den Echopixeln sowie die Bilder mit niedriger Qualität ohne den Echopixeln hinzugefügte interpolierte Werte erzeugt.

7. Das System gemäß einem der vorherigen Ansprüche, wobei der Bildprozessor des lokalen Ultraschall-Abtastgeräts zudem einen Videokompressionsprozessor umfasst.

8. Das System gemäß einem der vorherigen Ansprüche, wobei das lokale Ultraschall-Abtastgerät und das Ultraschallsystem jeweils eine Netzwerkkommunikationseinheit umfassen, die Steuerungs- und oder Bilddaten abruft oder sendet.

9. Eine Methode zum Durchführen eines Ultraschall-Abtastvorgangs von einem Remote-Standort aus, die folgende Schritte umfasst:
Abtasten eines Patienten Ultraschall mithilfe eines Ultraschall-Abtastgeräts am Standort des Patienten;
Senden der durch das Abtasten mit Ultraschall erfassten Ultraschallbilder mit hoher Qualität an den Remote-Standort;
Erzeugen von Ultraschallbildern mit niedriger Qualität als Reaktion auf ein vom Remote-Standort empfangenes Bildqualitätssteuerungssignal oder eine Standardeinstellung auf dem Ultraschall-Abtastgerät; und
Anzeigen der Ultraschallbilder mit niedriger Qualität am Standort des Patienten.

10. Die Methode gemäß einem der vorherigen Ansprüche, die das Abrufen einer Abtastanleitung am Remote-Standort sowie das Bereitstellen der Abtastanleitung am Standort des Patienten umfasst.

11. Die Methode gemäß einem der vorherigen Ansprüche, die das Anzeigen der Ultraschallbilder mit hoher Qualität auf einem Bildanzeigegerät am Remote-Standort umfasst.

12. Die Methode gemäß einem der vorherigen Ansprüche, wobei das Erzeugen von Ultraschallbildern mit niedriger Qualität vom Ultraschall-Abtastgerät durchgeführt wird.

13. Die Methode gemäß einem der vorherigen Ansprüche, die das Herstellen einer Sprachkommunikation zwischen dem Standort des Patienten und dem Remote-Standort umfasst.

14. Die Methode gemäß einem der vorherigen Ansprüche, die das Aktivieren des Ultraschall-Abtastgeräts für das Abtasten vom Remote-Standort aus umfasst.

## Revendications

1. Système de guidage à distance d'une procédure de balayage comprenant :
un dispositif de balayage à ultrasons local comprenant une sonde à ultrasons (10), un affichage d'image (12) et une
unité de communication d'image (30) conçue pour envoyer des images vers et recevoir des signaux de commande depuis un emplacement distant ;
un système à ultrasons (108),
lequel est situé à l'emplacement distant et conçu pour afficher des images reçues du dispositif de balayage à ultrasons local et envoyer des signaux de commande au dispositif de balayage à ultrasons local :
dans lequel le dispositif de balayage à ultrasons local comprend en outre un processeur d'image, lequel répond aux signaux de commande reçus du système à ultrasons et conçu pour générer des images de faible qualité de l'image à afficher sur l'affichage d'image et des images de haute qualité à envoyer au système à ultrasons à l'emplacement distant,
dans lequel l'affichage d'image est configuré pour afficher les images de qualité de l'image inférieure du dispositif de balayage à ultrasons local.

2. Système selon l'une quelconque des revendications précédentes, dans lequel les images de faible qualité de l'image comprennent une résolution d'image inférieure à celle des images de haute qualité.

3. Système selon l'une quelconque des revendications précédentes, dans lequel les facteurs de qualité pour les images de faible qualité et les images de haute qualité sont définis selon un paramètre par défaut dans le dispositif de balayage à ultrasons local.

4. Système selon l'une quelconque des revendications précédentes, dans lequel les facteurs de qualité pour les images de faible qualité et les images de haute qualité sont définis en fonction d'un signal de contrôle de qualité de l'image reçu du système à ultrasons situé à l'emplacement distant.

5. Le système selon l'une quelconque des revendications précédentes, dans lequel le dispositif de balayage à ultrasons local est conçu pour recevoir le guidage d'un expert à l'emplacement distant.

6. Le système selon l'une quelconque des revendications précédentes, dans lequel le processeur d'image du dispositif de balayage à ultrasons local comprend un convertisseur de balayage conçu pour générer les images de haute qualité présentant une interpolation complète des valeurs de pixels d'image entre des pixels d'écho et les images de faible qualité ne présentant aucune valeur interpolée ajoutée aux pixels d'écho.

7. Système selon l'une quelconque des revendications précédentes, dans lequel le processeur d'image du dispositif de balayage à ultrasons local comprend en outre un processeur de compression vidéo.

8. Système selon l'une quelconque des revendications précédentes, dans lequel le dispositif de balayage à ultrasons local et le système à ultrasons comprennent respectivement une unité de communication de réseau conçue pour envoyer et pour recevoir des données de commande et ou d'image.

9. Procédé de guidage d'une procédure de balayage par ultrasons à partir d'un emplacement distant comprenant :
le balayage par ultrasons d'un sujet à l'aide d'un dispositif de balayage par ultrasons sur l'emplacement d'un patient ;
l'envoi d'images par ultrasons de haute qualité acquises par balayage par ultrasons vers l'emplacement distant :
la génération des images par ultrasons de faible qualité en réponse à un signal de contrôle de la qualité de l'image reçu de l'emplacement distant ou à un réglage par défaut sur le dispositif de balayage à ultrasons ; et
l'affichage des images par ultrasons de faible qualité sur l'emplacement du patient.

10. Procédé selon l'une quelconque des revendications précédentes, comprenant l'obtention d'un guidage de balayage sur l'emplacement distant et la fourniture du guidage de balayage à l'emplacement du patient.

11. Procédé selon l'une quelconque des revendications précédentes, comprenant l'affichage des images par ultrasons de haute qualité sur un dispositif d'affichage d'image sur l'emplacement distant.

12. Procédé selon l'une quelconque des revendications précédentes, dans lequel la génération des images par ultrasons de faible qualité est effectuée par le dispositif de balayage par ultrasons.

13. Procédé selon l'une quelconque des revendications précédentes, comprenant l'établissement d'une communication vocale entre l'emplacement du patient et l'emplacement distant.

14. Procédé selon l'une quelconque des revendications précédentes, comprenant l'activation du dispositif de balayage par ultrasons destinée à un balayage depuis l'emplacement distant.
